# FASCICULE DE BREVET EUROPEEN

(11) **EP 0 587 866 B1**
(45) Date de publication et mention de la délivrance du brevet: **11.09.1996**
(21) Numéro de dépôt: 93908870.4
(22) Date de dépôt: 31.03.1993
(51) Int. Cl.: A61M 1/00

(54) **CANULE**
KANUELE
CANNULA

(30) Priorité: 02.04.1992 FR 9204149
(43) Date de publication de la demande: 23.03.1994
(73) Titulaire: GUIGNARD, Mireille, F-01630 Saint-Genis Pouilly (FR)
(72) Inventeur: GUIGNARD, Mireille, F-01630 Saint-Genis Pouilly (FR)
(74) Mandataire: Savoye, Jean-Paul
(86) Numéro de dépôt international: EP9300792
(87) Numéro de publication internationale: WO9319790

(56) Documents cités:
- FR-A- 2 321 902
- US-A- 2 541 691
- US-A- 4 692 153

## Description

La présente invention se rapporte à une canule pour l'évacuation d'un liquide de rinçage lors d'une intervention ou d'un examen endoscopique.

Il est bien connu que le bon déroulement des interventions chirurgicales sous endoscopies et plus particulièrement les arthroscopies par irrigation sous pression constante de sérum physiologique au travers d'un arthroscope, est lié à la bonne circulation du liquide de rinçage de l'arthroscope vers la canule qui donne la transparence, garante de la visibilité dans la cavité opératoire, en éliminant en permanence les débris ou le sang. L'arrêt de cette circulation, consécutif à l'obturation de la canule d'évacuation qui est raccordée à une aspiration, crée une opacité quasi immédiate du champ opératoire.

Dans une arthroscopie du genou, la position de la canule d'aspiration se situe de préférence dans une zone de la capsule articulaire où ne se trouvent pas le champ visuel de la caméra et les instruments chirurgicaux afin de ne pas entraver les gestes opératoires du chirurgien, ni réduire le champ visuel. De ce fait, la zone la plus fréquemment utilisée pour placer la canule est le cul-de-sac sous-quadricipital. Compte tenu de sa position, la canule est fréquemment isolée de la zone d'irrigation dans certaines positions du genou.

Dans ces conditions particulières et suivant l'importance de la synoviale, il peut arriver que le liquide d'irrigation ne puisse plus migrer de la poche ou se situe l'arthroscope à celle ou se situe la canule. A ce moment là, la circulation qui permet le rinçage est interrompue, le liquide se trouble rapidement et rend le champ opératoire opaque, empêchant toute intervention.

On a déjà proposé dans le US-2 541 691 un instrument de drainage qui présente à son extrémité avant une pluralité de languettes parallèles découpées dans un matériau élastique en feuille, enroulé ensuite selon un cylindre d'axe parallèle aux languettes, de manière que celles-ci s'écartent en direction de leurs extrémités.

Pour permettre d'introduire le drain dans une veine, les languettes sont ramenées manuellement vers l'axe du cylindre et retenues dans un capuchon fixé à l'extrémité d'une tige. Une fois le drain dans la veine on pousse axialement vers l'avant la tige de manière à dégager les languettes du capuchon.

L'écartement des languettes est obtenu sous l'effet de leur propre élasticité. Aucune force n'est appliquée sur elles pour les écarter. Un tel instrument ne permettrait pas de jouer le rôle d'écarteur en vue de rétablir la circulation de liquide entre la zone d'irrigation et la canule l'évacuation.

La présente invention se propose de remédier, au moins en partie, à cet inconvénient.

A cet effet, cette invention a pour objet une canule pour l'évacuation d'un liquide de rinçage lors d'une intervention ou d'un examen endoscopique, en particulier arthroscopique, selon la revendication 1.

L'avantage principal de l'invention provient du fait que la canule joue le rôle d'un écarteur quand elle est introduite dans le cul-de-sac sous-quadricipital. Elle maintient ainsi la capsule articulaire ouverte pour permettre la bonne circulation du liquide au travers de la cavité, entre l'arrivée dudit liquide au niveau de l'arthroscope et sa sortie au niveau de ladite canule.

D'autres avantages apparaîtront à l'aide de la description qui va suivre ainsi que des dessins annexés qui illustrent, schématiquement et à titre d'exemple, une forme d'exécution de la canule objet de l'invention.

La figure 1 est une vue en élévation latérale avec coupe partielle de cette canule, dans une première position de travail.

La figure 2 est une vue partielle de la figure 1, dans une seconde position.

La figure 3 est une vue latérale d'un genou en position d'extension avec une canule de l'état de la technique vue en coupe.

La figure 4 est une vue latérale du même genou en position de flexion.

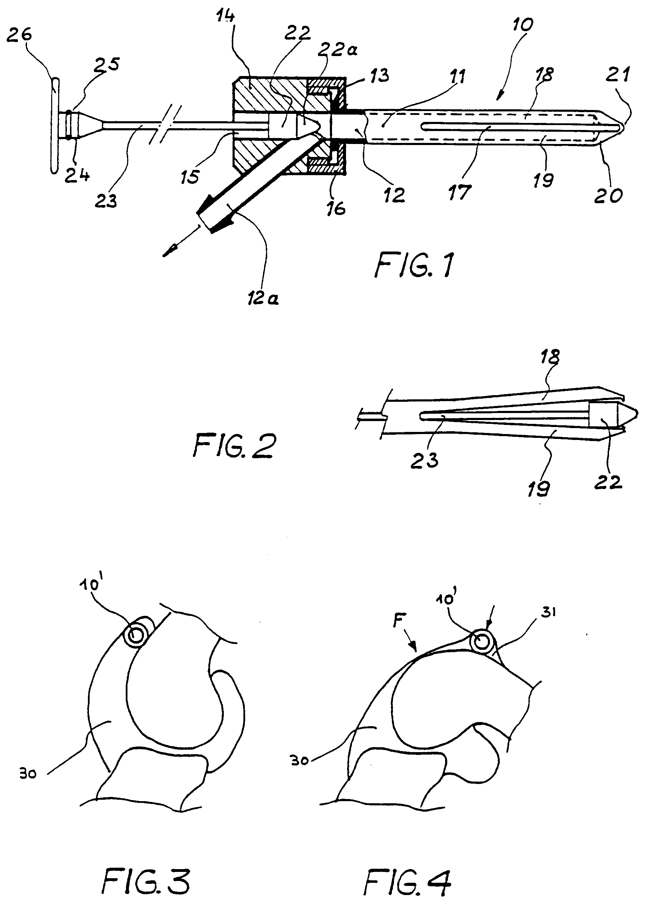

Les figures 3 et 4 illustrent un genou dans lequel on voit une canule 10' insérée dans le cul-de-sac sous-quadricipital antérieur 31, la zone d'intervention étant identifiée par la référence 30. Comme on le voit, alors que la canule communique avec la zone d'intervention 30 lorsque le genou est en extension (figure 3), la communication est interrompue à l'endroit de la flèche F lorsqu'il est en flexion, de sorte que le liquide de rinçage introduit au niveau de l'arthroscope (non représenté) se trouvant dans la zone d'intervention 30 ne peut plus être évacué par la canule 10' située dans la zone 31. La pompe alimentant l'arthroscope est munie d'un asservissement en pression, de sorte qu'elle s'arrête et que le liquide qui ne circule plus se trouble rapidement.

La canule 10 objet de l'invention, illustrée par les figures 1 et 2, comporte un élément tubulaire 11 dont le canal intérieur 12 sert de canal d'évacuation. Cet élément tubulaire 10 présente, à une extrémité, une collerette 13 qui permet de le fixer de façon amovible sur un élément de connexion 14 entre un canal 12a, formant un coude incliné par rapport à l'axe du canal 12 et destiné à être relié à une pompe d'aspiration (non représentée), et un passage 15 qui s'étend coaxialement au canal 12. Une bague de fixation 16, vissée sur l'élément de connexion 14, applique la collerette 13 contre une face de cet élément 14 dans laquelle débouche une extrémité du passage 15. Des moyens d'étanchéité (non représentés) sont prévus pour rendre étanche la fixation de l'élément tubulaire 11 sur l'élément de connexion 14.

L'élément tubulaire 11 comporte deux fentes 17 s'étendant dans un même plan diamétral et formant ainsi deux languettes 18 et 19 de part et d'autre du canal 12. Celui-ci s'arrête avant l'extrémité conique 20 de cet élément et un îlot de matière 21 est réservé pour former une liaison entre les extrémités respectives de ces languettes. De préférence, cet élément tubulaire 11 est en matière plastique, notamment en PVC.

Un piston 22, se terminant par une extrèmité conique d'écartement 22a, est engagé dans le passage 15. L'extrémité arrière de ce piston est solidaire d'une tige de manoeuvre 23 qui se termine par un second piston 24 muni d'un joint O-ring 25. Un organe de préhension 26, solidaire du piston 24, sert à actionner le piston 22 et son extrémité conique d'écartement 22a.

Lorsque la canule 10, illustrée par la figure 1, est mise en place en vue de l'intervention, par exemple, dans la position illustrée par les figures 3 et 4, on introduit d'abord l'élément tubulaire 11 dans la cavité appropriée. On pousse ensuite le piston 22 vers l'avant : son extrémité conique 22a rencontre une résistance en arrivant à l'extrémité de section réduite du canal 12 adjacent à l'extrémité libre de l'élément tubulaire. A ce moment, on tient d'une main l'élément de connexion 14 et, de l'autre, on pousse l'organe de préhension 26. La force ainsi développée par le cône 22a sur les extrémités libres des languettes 18,19 rompt la liaison formée par l'îlot de matière plastique 21 et permet alors d'écarter les languettes 18,19 l'une de l'autre comme illustré par la figure 2. Cet écartement des languettes 18 et 19 permet d'agir sur la paroi du sac sous-quadricipital en l'écartant de l'os pour rétablir la liaison entre les zones 30 et 31 en position de flexion du genou. Dans la position extrême antérieure du piston 22 illustrée par la figure 2, le piston 24 et son joint O-ring 25 sont ajustés dans le passage 15 et assurent ainsi l'étanchéité, de sorte que l'évacuation du liquide de rinçage s'effectue à travers le canal 12a que est de préférence incliné par rapport à l'axe commun du passage 15 et du canal 12.

Si, dans la position illustrée par la figure 2, le canal d'évacuation 12 venait à se boucher par obstruction d'un corps solide, il suffirait de retirer le piston 22 en arrière en tirant l'organe de préhension 26 pour extraire ce corps hors du canal et de ramener ensuite le piston 22 dans sa position d'écartement des languettes 18 et 19 en repoussant l'organe de préhension 26.

Bien entendu, la solution illustrée est un exemple d'exécution possible de l'invention et d'autres moyens pourraient être utilisés en particulier pour écarter les languettes 18 et 19. C'est ainsi que l'on pourrait imaginer de remplacer le piston 22 par un ballonnet gonflable et la tige 23 par un conduit d'alimentation dont une extrémité s'ouvrirait à l'intérieur du ballonnet et dont l'autre extrémité pourrait, par exemple, être reliée à un soufflet susceptible d'être actionné manuellement ou par une pédale pour gonfler l'élément d'écartement afin d'écarter les languettes 18 et 19 l'une de l'autre. Par ailleurs, le nombre des languettes 18 et 19 pourrait être supérieur à deux, par exemple trois ou quatre.

## Revendications

1. Canule pour l'évacuation d'un liquide de rinçage lors d'une intervention ou d'un examen endoscopique, en particulier arthroscopique comprenant une élément tubulaire (11) formant au moins en partie canal d'évacuation (12) dudit liquide de rinçage,
un passage (15) pour relier ledit canal (12) à l'extérieur,
un organe d'actionnement (22) et des moyens de commande (23, 26) de cet organe d'actionnement (22) susceptibles d'être manoeuvrés de l'extérieur dudit canal à travers ledit passage (15) pour déplacer longitudinalement ledit organe d'actionnement
des moyens d'étanchéité (25) pour permettre le passage étanche desdits moyens de commande de l'extérieur de la canule audit organe d'actionnement (22), au moins en position avancée de celui-ci, caractérisée par le fait
qu'au moins deux fentes latérales allongées (17) sont ménagées de part et d'autre de la partie du canal d'évacuation (12) formée par ledit élément tubulaire (11), partant d'une extrémité libre de cet élément tubulaire (11) et s'étendant sur une partie de sa longueur formant deux languettes (18, 19) solidaires de cet élément tubulaire (11) et
que ledit organe d'actionnement (22) est conformé pour appliquer auxdites languettes (18, 19) des forces tendant à les écarter l'une de l'autre dans la position avancée dudit organe d'actionnement.

2. Canule selon la revendication 1, caractérisée en ce que ledit élément tubulaire (11) est monté de façon amovible sur un élément de connexion entre ledit canal d'évacuation (12) et ledit passage (15).

3. Canule selon la revendication 2, caractérisée en ce que ledit élément tubulaire (11) et ledit passage (15) sont coaxiaux.

4. Canule selon la revendication 3, caractérisée en ce que la section droite dudit canal (12) est réduite au voisinage de l'extrémité libre desdites languettes, que l'organe d'actionnement (22) comporte un élément conique (22a), dont le sommet est tourné vers l'extrémité libre des languettes (18, 19) et dont la base a un diamètre sensiblement égal à celui dudit canal (12) et que les moyens de commande comportent une tige (23) solidaire dudit élément conique (22a) pour amener la base de celui-ci dans la portion de section droite réduite dudit canal (12).

5. Canule selon la revendication 4, caractérisée en ce que l'extrémité de ladite tige (23) opposée à celle solidaire dudit organe d'actionnement (22) se termine par un organe de manoeuvre (26) et par une surface de piston (24) associée à un joint O-ring (25) pour s'ajuster dans ledit passage (15).

6. Canule selon la revendication 1, caractérisée en ce que des moyens de liaison (21), susceptibles d'être rompus par la force engendrée par ledit organe d'actionnement (22), relient les extrémités libres desdites languettes (18, 19), l'une à l'autre.

7. Canule selon la revendication 6, caractérisée en ce que ledit élément tubulaire (11) est en matière plastique et que les moyens de liaison (21)sont formés par au moins un îlot de matière compris entre lesdites extrémités libres de languettes (18, 19).

## Claims

1. A cannula for evacuating a rinsing liquid during an endoscopic, and in particular arthroscopic, operation or examination, comprising a tubular element (11) that at least in part forms an evacuation conduit (12) for said rinsing liquid,
a passage (15) for connecting said conduit to the outside,
an actuating member (22) and control means (23, 26) for said actuating member (22) that are capable of being manoeuvred from outside said conduit through said passage (15) in order to longitudinally displace said actuating member,
sealing means (25) for enabling the sealed passage of said control means from outside the cannula to said actuating member (22), at least in forward position thereof, characterised in that
at least two lateral elongated slots (17) are provided on either side of the portion of the evacuation conduit (12) formed by said tubular element (11), beginning at one free of this tubular element (11) and extending over a portion of its length, forming two small tongues (18, 19) solid with this tubular element (11) and in that
said actuating member (22) is formed for exerting forces to said tongues (18, 19) leading to space apart from each other in the forward position of said actuating member.

2. The cannula of claim 1, characterised in that said tubular element (11) is detachably mounted on an element for connection between said evacuation conduit (12) and said passage (15).

3. The cannula of claim 2, characterised in that said tubular element (11) and said passage (15) are coaxial.

4. The cannula of claim 3, characterised in that the cross section of said conduit (12) is reduced in the vicinity of the free end of said tongues; that said actuating member (22) includes a conical element (22a), whose apex is oriented toward the free end of the tongues (18, 19) and whose base has a diameter substantially equal to that of said conduit (12); and that the control means include a rod solid with said conical element (22a) in order to move the base thereof into the portion of reduced cross section of said conduit (12).

5. The cannula of claim 4, characterised in that the end of said rod (23) opposite that solid with said actuating member (22) terminates in a manoeuvring device (26) and in a piston surface (24) associated with an O-ring (25), for adjustment within said passage (15).

6. The cannula of claim 1, characterised in that linking means (21) that can be broken by the force generated by said actuating member (22), join the free ends of said tongues (18, 19) to one another.

7. The cannula of claim 6, characterised in that said tubular element (11) is of plastic material, and that the linking means (21) are formed by at least one islet of material included between said free ends of the tongues (18, 19).

## Patentansprüche

1. Kanüle für die Entleerung einer Spülflüssigkeit während eines Eingriffs oder einer Untersuchung mittels Endoskopie und insbesondere mittels Arthroskopie, mit einem röhrenförmigen Element (11), das zumindest teilweise einen Entleerungskanal (12) für die benannte Spülflüssigkeit bildet,
mit einem Durchlass (15), um den benannten Kanal (12) mit aussen zu verbinden,
mit einem Betätigungsorgen (22) und Steuermitteln (23, 26) für dieses Betätigungsorgen (22), die sich von ausserhalb des benannten Kanals durch den benannten Durchlass (15) hindurch bedienen lassen, um das benannte Betätigungsorgen in seiner Längsrichtung zu verschieben,
und mit Dichtungsmitteln (25), um den abgedichteten Durchgang der benannten Steuermittel von ausserhalb der Kanüle zu dem benannten Betätigungsorgan (22) zumindest in dessen vorgeschobener Stellung zu ermöglichen, dadurch gekennzeichnet,
dass zumindest zwei gestreckte seitliche Schlitze (17) zu beiden Seiten des Teils des Entleerungskanals (12) angebracht sind, der durch das benannte röhrenförmige Element (11) gebildet wird, wobei diese Schlitze bei einem freien Ende dieses röhrenförmigen Elements (11) beginnen und sich entlang eines Teiles dieses Elements erstrecken, wodurch zwei Zungen (18, 19) gebildet werden, die mit diesem röhrenförmigen Element (11) fest verbunden sind, und
dass das benannte Betätigungsorgen (22) so gestaltet ist, dass Kräfte auf die benannten Zungen (18, 19) ausgeübt werden, die darauf abzielen, in der vorgeschobenen Stellung des benannten Betätigungsorgans diese Zungen auseinanderzuspreizen.

2. Kanüle gemäss Anspruch 1, dadurch gekennzeichnet, dass das benannte röhrenförmige Element (11) abnehmbar auf einem Verbindungselement zwischen dem benannten Entleerungskanal (12) und dem benannten Durchlass (15) angebracht ist.

3. Kanüle gemäss Anspruch 2, dadurch gekennzeichnet, dass das benannte röhrenförmige Element (11) und der benannte Durchlass (15) koaxial sind.

4. Kanüle gemäss Anspruch 3, dadurch gekennzeichnet, dass der Querschnitt des benannten Kanals (12) in Nachbarschaft des freien Endes der benannten Zungen verengt ist, dass das Betätigungsorgan (22) ein kegelförmiges Element (22a) umfasst, dessen Spitze zum freien Ende der Zungen (18, 19) hingewendet ist und dessen Grundfläche in ihrem Durchmesser im wesentlichen dem benannten Kanal (12) gleicht, und dass die Steuermittel eine Stange (23) umfassen, die mit dem benannten kegelförmigen Element (22a) fest verbunden ist, um die Grundfläche dieses Elements in dem verengten Abschnitt des benannten Kanals (12) zu führen.

5. Kanüle gemäss Anspruch 4, dadurch gekennzeichnet, dass das Ende der benannten Stange (23), das dem mit dem benannten Betätigungsorgan (22) fest verbundenen Ende entgegengesetzt ist, in ein Steuerorgan (26) und in eine Kolbenfläche (24) ausläuft, auf der ein Dichtungsring (25) sitzt, um in den benannten Durchlass (15) eingepasst zu werden.

6. Kanüle gemäss Anspruch 1, dadurch gekennzeichnet, dass Verbindungsmittel (21), die sich durch die von dem benannten Betätigungsorgan (22) ausgeübte Kraft aufbrechen lassen, die freien Enden der benannten Zungen (18, 19) miteinander verbinden.

7. Kanüle gemäss Anspruch 6, dadurch gekennzeichnet, dass das benannte röhrenförmige Element (11) aus Kunststoff besteht und dass die Verbindungsmittel (21) aus zumindest einer zwischen den benannten freien Enden der Zungen (18, 19) eingeschlossenen Stoffbrücke bestehen.
